# EUROPÄISCHE PATENTANMELDUNG

(11) **EP 1 952 797 A1**
(43) Veröffentlichungstag der Anmeldung: **06.08.2008**
(21) Anmeldenummer: 08001348.5
(22) Anmeldetag: 24.01.2008
(51) Int. Cl.: A61K 8/44, A61Q 19/08

(54) **Verwendung einer kosmetischen Zusammensetzung enthaltend NMDA-Rezeptor-Agonisten zur kosmetischen Behandlung der Haut**

(30) Priorität: 24.01.2007 DE 102007003582
(71) Anmelder: Dr. August Wolff GmbH & Co. KG Arzneimittel, 33611 Bielefeld (DE)
(72) Erfinder: Fischer, Matthias, 06120 Halle (DE)
(74) Vertreter: Grünecker, Kinkeldey, Stockmair & Schwanhäusser Anwaltssozietät

(57) **Zusammenfassung**

Die vorliegende Erfindung betrifft die Verwendung einer kosmetischen Zusammensetzung enthaltend NMDA-Rezeptor-Agonisten zur kosmetischen Behandlung der Haut.

## Beschreibung

Die vorliegende Erfindung betrifft die Verwendung von NMDA-Rezeptor-Agonisten zur kosmetischen Behandlung der Haut.

Die Haut stellt die äußere Begrenzung des Individuums zu seiner Umgebung dar und ist eine Barriere gegen eine Vielzahl von schädlichen Umwelteinflüssen. Ihr äußerster Anteil, die Epidermis, ist ein geschichtetes Plattenepithel ektodermaler Herkunft, das zu ca. 90% aus Keratinozyten besteht, die aus dem sich stetig regenerierenden Stratum basale hervorgehen. Ihm folgt das 2-6 Zelllagen dicke Stratum spinosum, in dem eine allmähliche Zunahme des Zellvolumens zu beobachten ist. In dem sich anschließenden Stratum granulosum beschleunigt sich die Differenzierung der Keratinozyten, die mit dem Verschwinden von funktionellen Zellorganellen und dem Beginn der terminalen Verhornung einhergeht. Die Zellen enthalten zunehmend basophile Keratohyalingranula und sind deutlich abgeflacht. Als oberflächlichste Schicht der Epidermis schließt sich dann das Stratum corneum (Homschicht) an, das aus flachen, fest gepackten, kernlosen Homzellen besteht, die dicht mit Tonofilamenten und einer amorphen Matrix gefüllt sind. Die Umwandlung von vitalen Keratinozyten des oberen Stratum granulosum in die Hornschicht ist ein abrupter Vorgang, der sich lediglich über Stunden erstreckt.

Zur Aufrechterhaltung der Funktion der Epidermis ist eine ausreichende Proliferation der Keratinozyten mit nachfolgender kontrollierter orthologer Differenzierung und Verhornung von entscheidender Bedeutung. Diese Vorgänge unterliegen einem stetigen Einfluss von Wachstumsfaktoren, Zytokinen, Fettsäuren und Ceramiden. Aminosäuren kommen hierbei vielfältige Aufgaben bei der Aufrechterhaltung der orthologen Entwicklung des Keratinozyten zu. Aminosäuren sind organische Verbindungen, die zumindest aus einer Carboxyl- und Aminogruppe bestehen. In der Epidermis wirken sie als:
- Bausteine der Proteinbiosynthese
- Transmitter (Botenstoffe)
- Einflussfaktoren des Säure-Base-Haushalts

Eine für diese komplexen Wirkungen besonders wichtige Aminosäure ist L-Glutamat, die alle drei genannten Punkte beeinflusst. L-Glutamat ist eine nicht essentielle Aminosäure, die im Zellstoffwechsel eine große Rolle spielt. Sie ist einerseits Baustein der Proteinbiosynthese, gleichzeitig aber auch an Stoffwechselvorgängen wie beispielsweise dem Zitronensäurezyklus und der Bindung von Ammoniak beteiligt.

Als Transmitter ist L-Glutamat bei Nervenzellen seit langem bekannt. Es ist der wichtigste exzitatorische Transmitter im Nervensystem der Vertebraten, der seine Wirkung über die Bindung an verschiedenen ionotropen und metabotropen Glutamatrezeptoren entfaltet. Metabotrope Glutamatrezeptoren erhöhen als G-Protein-gekoppelte Rezeptoren entweder über Gq Phospholipase C die intrazelluläre Calciumkonzentration (Rezeptoren der Gruppe I) oder führen über Gi zu einer Verminderung von intrazellulärem cAMP (Rezeptoren der Gruppen II + III) [Kew und Kemp, Psychopharmacology (Berl). 2005; 179: 4-29]. Die ionotropen Glutamatrezeptoren hingegen sind ligandengesteuerte, nicht-selektive, postsynaptisch gelegene, transmembranöse Kationenkanäle [Kew und Kemp, Psychopharmacology (Berl). 2005; 179: 4-29]. Neben L-Glutamat sind noch eine Reihe spezifischer Agonisten bekannt, die selektiv an verschiedenen ionotropen Glutamatrezeptoren binden. Nach diesen selektiven Agonisten werden die ionotropen Glutamatrezeptoren weiter in *AMPA-* (Amino-3-hydroxy-5-methyl-4-isoxazolpropionat), *Kainat-* und *NMDA-Rezeptoren* (N-Methyl-D-Aspartat) unterteilt [Kew und Kemp, Psychopharmacology (Berl). 2005; 179: 4-29]. Die durch Glutamatrezeptoren ausgelösten Effekte sind vielfältig. Neuere Studien deuten jedoch darauf hin, dass L-Glutamat auch als Botenstoff für Keratinozyten bedeutsam ist. Bei diesen Rezeptor-gestützten Funktionen ist der Effekt am NMDA-Rezeptor des Keratinozyten von wesentlicher Bedeutung.

NMDA-Rezeptoren gehören zur Familie der ionotropen Glutamatrezeptoren und bestehen aus einer konstanten Untereinheit (R1) sowie den variablen Untereinheiten NR2A bis NR2D, NR3A und NR3B. NMDA-Rezeptoren haben eine tetramere Struktur. Der Rezeptor ist ein lonenkanal, der als Transmembranprotein Ionen ermöglicht, die Zellmembran zu durchqueren. Im Unterschied zu dem aktiven Transport mit lonen-Transportern erfolgt der Transport in Ionenkanälen passiv, dem elektrochemischen Gradienten entsprechend. Im Falle von NMDA-Rezeptoren erfolgt die Öffnung (=Aktivierung) des Kanals durch einen Liganden (Botenstoff). Der natürliche Ligand am NMDA-Rezeptor ist L-Glutamat. Nach seiner Öffnung ist der NMDA-Rezeptor vorrangig für Calcium-lonen durchgängig. Für die Funktion des Rezeptors, seine Stimulation durch Agonisten und seine Hemmung durch Antagonisten sind dabei o.g. variablen Untereinheiten von besonderer Bedeutung. Unter Ruhebedingungen ist der NMDA-Rezeptor geschlossen und durch Magnesium-lonen blockiert (spannungsabhängiger Magnesiumblock bei negativem Membranpotenzial). Das Ausmaß des Magnesium-Effekts ist jedoch vom Typ des NMDA-Rezeptors abhängig und bei Keratinozyten aufgrund des vorhandenen Subtyps NMDAR2D als gering einzustufen. Erst nach der Stimulation durch L-Glutamat oder einen Agonisten, wie N-Methyl-D-Aspartat, kommt es zur Öffnung des Kanalproteins mit nachfolgendem intrazellulärem Calciumeinstrom.

Die Existenz von Glutamatrezeptoren vom Typ der N-Methyl-D-Aspartat-Rezeptoren (NMDA-Rezeptoren) in Keratinozyten konnte nachgewiesen werden (Fischer et al. Arch. Dermatol. Res. 2004, 296). Dabei fand sich in der gesunden Epidermis eine Expression von NMDA-Rezeptoren in allen vitalen Schichten der Epidermis mit besonderer Betonung des Stratum granulosum. Dieses Verteilungsmuster korreliert dabei sehr gut mit dem bekannten intraepidermalen Calciumgradienten, der durch einen abrupten Anstieg im Stratum granulosum gekennzeichnet ist (Menon und Elias: Arch. Dermatol. 1991; 127: 57-63). Dieser Calcium-Peak wird als wesentliches Signal für die Differenzierung der Keratinozyten angesehen. In weiterführenden Untersuchungen zur Funktion keratinozytärer NMDA-Rezeptoren fanden sich überraschenderweise Hinweise auf eine differenzierungsfördernde Wirkung. So führte die Blockade des NMDA-Rezeptors mit dem selektiven Antagonisten MK-801 bei kultivierten Keratinozyten (HaCaT-Zellen) zur verminderten Expression der Differenzierungsmarker Filaggrin und Cytokeratin 10 (Fischer et al. Arch. Dermatol. Res. 2004, 296, 157-162; Fischer et al. Experimental Dermatology 2004, 13, 512-519).

Jedoch beschreiben Fuziwara et al. eine verzögerte Wiederherstellung der epidermalen Barriere durch den NMDA-Agonisten N-Methyl-D-Aspartat und L-Glutamat bei Untersuchungen an Nacktmäusen (Fuziwara et al. J. Invest. Dermatol. 2003, 120, 1023-1029) und interpretierten dies als Ausdruck einer vermehrten Proliferation von Keratinozyten nach Stimulation des NMDA-Rezeptors. Dies lässt eine Anwendung von NMDA-Rezeptor-Agonisten als Kosmetikum als problematisch erscheinen.

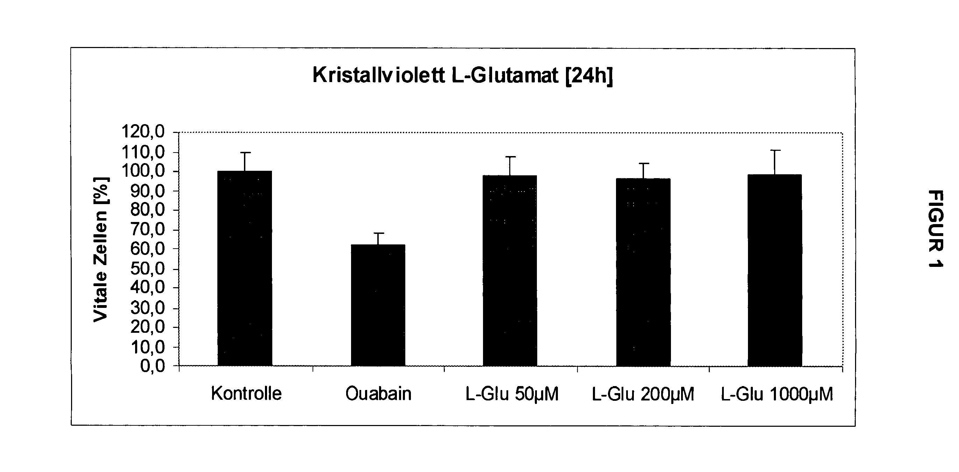

Diese Einschätzung konnte in eigenen Untersuchungen nicht belegt werden. Hierfür wurde die Kristallviolettmethode nach Gillies (Gillies et al., Anal. Biochem. 1986, 159, 109-113) verwandt, die sowohl eine Beurteilung des zytotoxischen Potenzials von Chemikalien, als auch deren Einfluss auf die Proliferation zulässt. Hierbei fanden sich keine Hinweise auf ein zytotoxisches oder proliferationssteigerndes Potenzial von NMDA (Fischer et al. Experimental Dermatology 2004, 13, 512-519) als auch von L-Glutamat (Figur 1).

### Figurenbeschreibung

Figur 1: Kristallviolett-Test nach Gillies von L-Glutamat bei kultivierten normalen humanen epidermalen Keratinozyten (Applikationsdauer 24h). Kein Nachweis einer zytotoxischen Reaktion oder der Induktion einer Proliferation. Als Positivkontrolle wurde Ouabain eingesetzt, das bekanntermaßen einen zytotoxischen Effekt besitzt.

Aufgabe der vorliegenden Erfindung ist es, eine Zusammensetzung zur Verwendung in der kosmetischen Behandlung der Haut zur Verfügung zu stellen. Dabei soll die Physiologie gesunder Haut erhalten und unterstützt werden. Weiterhin soll bei bereits vorhandenen Beeinträchtigungen ein unvorteilhaftes Erscheinungsbild der Haut verbessert und ein glattes Hautbild erzeugt werden. Schließlich darf die kosmetische Zusammensetzung keine Nebenwirkungen hervorrufen.

Diese Aufgabe wird durch die Verwendung einer kosmetischen Zusammensetzung gelöst, die zur nicht-therapeutischen Behandlung bzw. kosmetischen Behandlung der Haut eingesetzt wird, wobei die Zusammensetzung mindestens einen N-Methyl-D-Aspartat-Rezeptor-Agonisten enthält. In einer bevorzugten Ausführungsform enthält die Zusammensetzung zusätzlich mindestens einen natürlich vorkommenden N-Methyl-D-Aspartat-Rezeptor-Co-Agonisten. Ebenso bevorzugt sind Zusammensetzungen, welche mindestens ein Zn²⁺-chelatisierendes Agens enthalten. Besonders bevorzugt sind Zusammensetzungen, welche sowohl mindestens einen natürlich vorkommenden N-Methyl-D-Aspartat-Rezeptor-Co-Agonisten als auch mindestens ein Zn²⁺-chelatisierendes Agens enthalten.

Die erfindungsgemäß verwendete Zusammensetzungen enthält vorzugsweise mindestens ein Ca²⁺-Ionen spendendes Agens.

Erfindungsgemäß erfolgt an den kosmetisch zu behandelnden Stellen der Haut eine Wiederherstellung bzw. der Erhalt der Homöostase des Keratinozyten. Dieser Effekt wird durch die entsprechende Applikation der beanspruchten Zusammensetzung erzielt. Hierbei werden den Keratinozyten gleichzeitig Bausteine der Proteinbiosynthese zur Verfügung gestellt und der pH-Wert der Hautoberfläche normalisiert. Ferner bewirken die eingesetzten natürlich vorkommenden NMDA-Rezeptor-Agonisten eine Aktivierung des NMDA-Rezeptors. Dies erfolgt durch die Bindung des Wirkstoffs an extrazelluläre Stellen des NMDA-Rezeptors. Unter der Aktivierung versteht man den Vorgang der Öffnung des transmembranösen Calciumkanals, welcher ein integraler Bestandteil des NMDA-Rezeptors ist. Hierdurch können Ca²⁺-lonen aus dem extrazellulären Raum in die Zellen eindringen, wodurch eine Erhöhung der intrazellulären Calciumkonzentration bewirkt wird. Die dadurch bewirkte Erhöhung der intrazellulären Calciumkonzentration fördert die Differenzierung der Keratinozyten.

Hierfür werden Zusammensetzungen aus natürlich vorkommenden Aminosäuren und Aminosäurederivaten verwendet, welche zusätzlich ein physiologisch wirksames Ca²⁺-spendendes Agens enthalten. Dieses dient der Sicherung einer ausreichend hohen extrazellulären Calcium-Konzentration in der Epidermis, so dass ausreichend Calcium durch den aktivierten NMDA-Rezeptor in die Zellen strömen kann.

Erfindungsgemäß können grundsätzlich alle natürlich vorkommenden Substanzen als NMDA-Rezeptor-Agonisten verwendet werden, die eine ausreichende Selektivität für den Rezeptor aufweisen bei gleichzeitig geringer Toxizität. Beispiele für NMDA-Rezeptor-Agonisten, die erfindungsgemäß verwendet werden können, sind L-Glutamat, L-Aspartat, N-Methyl-D-Aspartat, einem Chinolat, einem Homocysteat, D-Aspartat, D-Glutamat, trans-1-Amino-cyclobutan-1,3-dicarbonsäure, (±)-1-Aminocyclo-pentan-cis-1,3-dicarbonsäure, 2-Carboxy-3-carboxymethylchinolin, L-Cystein-sulfinsäure, 3-Carboxy-methylpyridine-2-carbonsäure, α-Amino-(3-hydroxy-5- isoxazolyl)essigsäure, 4-Methoxy-7-nitroindolinyl-caged-L-glutamat, (S)-α-Amino-2,3-dihydro-4-methoxy-7-nitro-o-oxo-1H-indole-1-pentansäure, Pyridin-2,3-di-carbonsäure, (RS)-(Tetrazol-5-yl)glycin und Derivaten davon. Bevorzugt sind L-Glutamat, L-Aspartat und N-Methyl-D-Aspartat.

Weiterhin umfasst der Ausdruck "NMDA-Rezeptor-Agonist" erfindungsgemäß auch Verbindungen, die erst nach Verwendung der erfindungsgemäßen Zusammensetzung und der darauf folgenden Aufnahme in die Körperzellen zu den eigentlichen NMDA-Rezeptor-Agonisten umgewandelt werden. Diese Verbindungen werden im folgenden mit dem Begriff Vorstufen bezeichnet. Beispielhaft für diese Vorstufen kann α-Ketoglutarat genannt werden, das durch Metabolisierung in den NMDA-Rezeptor-Agonisten Glutamat umgewandelt wird.

Auf der NR1 Untereinheit des NMDA-Rezeptors befindet sich eine extrazelluläre Glycin-Co-Agonist-Bindungsstelle. Die Bindung eines entsprechenden Co-Agonisten an dieser Stelle verstärkt die Wirkung des NMDA-Rezeptor-Agonisten am gleichnamigen Rezeptor hinsichtlich seiner Aktivierung. Bevorzugt ist daher die Verwendung einer kosmetischen Zusammensetzung, welche sowohl einen NMDA-Rezeptor-Agonisten als auch einen NMDA-Rezeptor Co-Agonisten enthält, wobei letzterer an die Glycin-Co-Agonist Bindungsstelle bindet. Dadurch wird die Wirkung der kosmetischen Zusammensetzung hinsichtlich der Erhöhung der intrazellulären Calciumkonzentration insgesamt verstärkt.

Erfindungsgemäß kommen als NMDA-Rezeptor Co-Agonist alle Substanzen in Frage, die eine ausreichende Selektivität an dem Rezeptor aufweisen bei gleichzeitig geringer Exotoxizität, bzw. Toxizität bei systemischen Behandlungs-Varianten. Hierzu zählen unter anderem Glycin, D-Serin, L-Alanin, D-Cycloserin, R(+)-3-amino-1-hydroxypyrrolid-2-on, R(+)-cis-β-methyl-3-amino-1-hydroxy-pyrrolid-2-on, einem Polyamin, und Derivaten davon. Bevorzugt wird erfindungsgemäß Glycin als Co-Agonist eingesetzt.

Auf der NR2 Untereinheit des NMDA-Rezeptors befindet sich eine extrazelluläre Bindungsstelle für Zn²⁺-Ionen. Die Bindung von Zink-Ionen an dieser Stelle bewirkt eine Inhibierung der Aktivität des NMDA-Rezeptors, wodurch der Fluß an Calcium-Ionen in die Zelle gehemmt wird. Extrazelluläre Zn²⁺-Ionen, welche an den NMDA-Rezeptor binden können, mindern daher die Wirkung der erfindungsgemäß verwendeten kosmetischen Zusammensetzung. Besonders bevorzugt ist daher die Verwendung einer kosmetischen Zusammensetzung, welche einen NMDA-Rezeptor-Agonisten, einen NMDA-Rezeptor-Co-Agonisten und ein Zn²⁺-chelatisierendes Agens enthält.

Als Zn²⁺-chelatisierendes Agens werden erfindungsgemäß vorzugsweise Dithiotreithol, Glutathion, CaEDTA oder Derivate davon verwendet.

In einer bevorzugten Ausführungsform wird mindestens ein N-Methyl-D-Aspartat-Rezeptor-Agonist mit mindestens einem N-Methyl-D-Aspartat-Rezeptor-Co-Agonisten kombiniert. Dabei kann jeder der oben aufgeführten N-Methyl-D-Aspartat-Rezeptor-Agonisten mit jedem der oben aufgeführten N-Methyl-D-Aspartat-Rezeptor-Co-Agonisten beliebig kombiniert werden. In einer weiteren bevorzugten Ausführungsform wird mindestens ein N-Methyl-D-Aspartat-Rezeptor-Agonist mit mindestens einem Zn²⁺-chelatisierenden Agens kombiniert. Dabei kann jeder der oben aufgeführten N-Methyl-D-Aspartat-Rezeptor-Agonisten mit jedem der oben aufgeführten Zn²⁺-chelatisierenden Agentien beliebig kombiniert werden.

Für die Applikation kommen alle kosmetische Darreichungsformen in Betracht, welche die gewünschte Wirkung herbeiführen, vorausgesetzt die sonstigen Bestandteile der Zusammensetzung gehen keine unerwünschten Wechselwirkungen oder Reaktionen mit der Haut oder den Wirkstoffen selbst ein. Bevorzugt ist die topische Anwendung, wobei die kosmetische Zusammensetzung beispielsweise in Form einer halbfesten Zusammensetzung, wie einer Salbe, einer Creme oder eines Gels, oder in Form einer Lösung verabreicht werden kann.

Bei den halbfesten Zubereitungen kann es sich beispielsweise um hydrophobe, wasseraufnehmende oder hydrophile Salben handeln. Cremes umfassen mehrphasige Zubereitungen und können hydrophob, amphiphil oder hydrophil sein. Dagegen sind Gele durch Quellmittel gelierte Flüssigkeiten, die nach Auftragung durch das Verdunsten des Wassers eine kühlende und lindernde Wirkung aufweisen. Dabei bilden sie ein Gerüst, in dem die Flüssigkeit samt dem darin gelösten Wirkstoff an der Oberfläche immobilisiert wird. Gele können dabei sowohl hydrophob (Oleogele) als auch hydrophil (Hydrogele) wirken. In bestimmten Fällen ist die Verwendung einer Paste angezeigt, die zwar eine ähnliche Grundlage wie die Salben besitzt, jedoch zusätzlich einen großen Anteil an fein dispergiertem Pulver enthält.

Wird die kosmetische Zusammensetzung als Lösung verwendet, kann die Lösung in Ampullen gelagert werden, die die Wirkstoffe in hochkonzentrierter Form enthalten.

Die erfindungsgemäß verwendete kosmetische Zusammensetzung enthält den NMDA-Rezeptor-Agonisten üblicherweise in einer Konzentration von 0,1 µM - 1 M in den flüssigen Zubereitungen bzw. in einer Konzentration von 0,001 % - 30 % in den halbfesten Zubereitungsformen.

Bevorzugt enthält die kosmetische Zusammensetzung zusätzlich einen NMDA-Rezeptor-Co-Agonisten in einer Konzentration von 1 nM - 1 M in den flüssigen Zubereitungen bzw. in einer Konzentration von 0,001 % - 30 % in den halbfesten Zubereitungsformen.

Noch bevorzugter sind Ausführungen, welche zusätzlich ein Zn²⁺-chelatisierendes Agens in einer Konzentration von 1 nM - 1 M in den flüssigen Zubereitungen bzw. in einer Konzentration von 0,001 % - 30 % in den halbfesten Zubereitungsformen enthalten.

Besonders bevorzugt sind Ausführungen, welche zusätzlich ein Ca²⁺-lonen spendendes Agens in einer auf das Calcium bezogenen Konzentration von 1 nM - 1 M in den flüssigen Zubereitungen bzw. in einer Konzentration von 0,001 % - 30 % in den halbfesten Zubereitungsformen enthalten.

Die erfindungsgemäße Verwendung der kosmetischen Zusammensetzung kann bevorzugt in Kombination mit weiteren aktiven Substanzen erfolgen, wie Vitamine und Flavonoide, Lichtschutzfiltern, feuchtigkeitsspendenden Wirkstoffen oder anderen aktiven Bestandteilen. Zusätzlich können die bei der erfindungsgemäßen Verwendung hergestellten Zusammensetzungen übliche Bestandteile enthalten, wie sie im Folgenden dargelegt sind.

Die Zusammensetzungen können durch den Fachmann bekannte Herstellungsverfahren hergestellt werden. Die dazu notwendigen Vorrichtungen und Prozessparameter sind dem Fachmann bekannt. So können beispielsweise N-Methyl-D-Aspartat-Rezeptor-Agonisten oder eine N-Methyl-D-Aspartat-Rezeptor-Agonisten enthaltende Zubereitung mit einer Basiszusammensetzung für gewünschte Formulierungen (Creme, Shampoo etc.) vermischt und so die erfindungsgemäße Formulierung erhalten werden.

In der fertigen kosmetischen Formulierung können die NMDA-Antagonisten mit allen bekannten und üblichen Hilfsstoffen, Emulgatoren, Stabilisatoren, Konservierungsstoffen und/oder Lichtschutzfilter kombiniert werden. Ebenso vorstellbar sind weitere biologisch wirksame Stoffe oder Zubereitungen, wie z.B. Aminosäuren, Oligo- oder Polypeptide, natürlicher oder biotechnologischer Herkunft, Vitamine, Saccharide, Pflanzenextrakte sowie deren gereinigte Fraktionen, enthaltend Polyphenole, Flavonoide, Terpenoide und andere sekundäre Pflanzenbegleitstoffe.

Nachfolgende Aufzählung einzelner Stoffe ist beispielhaft, benennt lediglich bevorzugte Ausführungsformen und erhebt keinen Anspruch auf Vollständigkeit. Sie soll den Stoffcharakter der möglichen Kombinationen zeigen, die sich dem Produktentwickler eröffnen. Reinigungsprodukte, die als Gel, Aerosol- oder Non Aerosolschaum appliziert werden, setzen sich vorzugsweise aus einer Kombination anionischer Tenside, wie Laurylethersulfat, Laurylsulfat, Ethercarboxylate mit variablem Ethoxylierungsgrad, Ethersulfonate auf der Grundlage von Aminosäuren, Zuckern und Fettalkoholen zusammen. Diese wer den mit amphoteren Tensiden wie Cocamidopropyl Betain, Tauraten kombiniert. Zur Verbesserung des Hautgefühls und der Rückfettung können nichtionische Tenside auf Zucker und Lipidbasis eingesetzt werden.

Weitere Stoffbeispiele für die jeweiligen Produktgrundlagen sind: Acylglutamate, z.B. Natrium Acylglutamat, Na-Laurylglutamat, Na-Capryl/Capringlutamat, DI-TEApalmitoylaspartat, Acylpeptide wie Palmitoyl-hydrolysiertes Milchprotein, Sojaprotein, Collagen oder Keratin. Cocosfettsäurekondensate der genannten Proteinquellen. Sarcosinate, z.B. Myristoylsarcosin, TEA-Lauroylsarcosin, Na-Lauroylsarcosin, Na-Cocoylsarcosin, Taurate der Fettsäuren mit einer Kettenlänge von C10 bis C20, gesättigt oder ungesättigt, Acyllysinate, -alaninate oder -glycinate des zuvor beschriebenen Fettsäurespektrums.

Co-Tenside wie Cocamidopropyl Betaine, Decyl Polyglycoside, Dodecyl Polyglycoside, Disodium Laureth Sulfosuccinate, Trilaureth 4-Phosphate, Di/Mono Sodium Cocoamphoacetate.

Als Co-Emulgatoren oder Rückfetter kommen in Frage:
PEG-nn hydriertes Ricinusöl, PEG-6 Caprylsäure/Caprinsäureglyceride, Glyceryloleat im Gemisch mit Propylenglycol, PEG-9-Stearat, Ceteth-2, Ceteth-20, Po1ysorbat 60, Glycerylstearat im Gemisch mit PEG100, Glycerylmyristat, Glyceryllaurat, PEG-40-Sorbitanperoleat, Laureth-4, Ceteareth-3, Isostearylglycerylether, Cetylstearylalkohol im Gemisch mit Natrium Cetylstearylsulfat, Lameth-23, Steareth-2, Glycerylstearat im Gemisch mit PEG-3() Stearat. PEG-40-Stearat, Glycol Distearat, PEG-22-Dodecy1 Glyco1, Po1yglyceryl-2 PEG4-Stearat, Ceteareth-20, Methylglucosesesquistearat, Steareth-10, PEG-20-Stearat, Steareth-2 im Gemisch mit PEG-8 Distearat, Steareth-21, Steareth-20, Isosteareth-20, PEG-45 Dodecyglycol-Copolymer, Methoxy-PEG-22 Dodecylglycol-Copolymer, PEG-40-Sorbitanperol eat, PEG-40-Sorbitanperisostearat, PEG-20-Glycerylstearat, PEG-20-Glycerylstearat, PEG-8- Bienenwachs, Polyglyceryl-2-laurat, Isostearyldiglycerylsuccinat, Stearamidopropyl-PG-dimoniumchloridphosphat, Glycerylstearat SE, PEG-20-Methylglucosesesquistearat, Ceteareth-12, Glycerylstearatcitrat, Cetylphosphat, Sorbitansesquioleat, Trilaureth-4-Phosphat, Polyglycerylglucosedistearat, Kaliumcetylphosphat, Isosteareth-10, Polyglyceryl-2-sesquiisostearat, Ceteth-10, Oleth-20, Isoceteth-20, Glycerylsterat im Gemisch mit Ceteareth-20, Ceteareth-12, Cetylstearylalkohol und Cetylpalmitat, Cetylstearylalkohol im Gemisch mit PEG-20.Stearat, PEG-30-Stearat, PEG-40-Stearat, PEG-100-Stearat.

Es ist dem Fachmann natürlich bekannt, dass anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel. Stabilisatoren, Alkohol, Wasser, Salze, antimikrobiell, proteolytisch oder keratolytisch wirksame Substanzen usw. Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tages- oder Nachtcreme usw. Es ist gegebenenfalls möglich und vorteilhaft, die Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindung, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescremes gewöhnlich UV-A- bzw. UV-B-Filtersubstanzen eingearbeitet.

Vorteilhaft können erfindungsgemäße Zubereitungen Substanzen enthalten, die UV-Strahlung im UVB- Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen beträgt

Die UVB-Filter können öllöslich oder wasserlöslich sein. Als Öllösliche Substanzen sind z. B. zu nennen:
- 3-Benzylidencampher und dessen Derivate, z. B. 3-(4-Methylbenzyliden)campher,
- 4-Aminobenzoesäure Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexy1)ester, 4-Metboxyzimtsäureisopentylester;
- Ester der Salicylsäure, vorzugsweise Salicylsäure(2-ethylhexyl)ester, Salicylsäure.(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-metboxybenzophenon;
- Ester der Benzalmalonsäure, vorzugsweise 4-Methoxybenzalmalonsäuredi(2-ethylhexyl)ester; -2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z. B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z. B. 4-(-Oxo-3-bornylidenmethyl)benzolsulfonsäure, 2-Methyl-5-(2-oxo-3-bomylidenmethyl)sulfonsäure und ihre Salze.

Die Liste der genannten UVB-Filter, die erfindungsgemäß Verwendung finden können, ist nicht limitierend aufzufassen.

Es kann auch von Vorteil sein, in Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)-Propan-1,3-dion und um 1-Phenyl-3-(4.-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die kosmetischen und dermatologischen Zubereitungen können kosmetische Wirk-, Hilfs- und/oder Zusatzstoffe enthalten, wie sie üblicherweise in solchen Zubereitungen verwendet. werden, z. B. Antioxidationsmittel, Konservierungsmittel, Bakterizide, Parfüme, Substanzen zum Verbinden des Schäumens, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polyglycole, Schaumstabilisatoren, Elektrolyte, Organische Lösungsmittel oder Silikonderivate.

Es ist vorteilhaft, den Zubereitungen im Sinne der vorliegenden Erfindung weitere antirritative oder antientzündliche Wirkstoffe zuzugeben, insbesondere Batylalkohol ([alpha]-Octadecylglyccrylether), Selachylalkohol, Chimylalkohol ([alpha]-Hexadecylglycerylether), Bisabolol und/oder Panthenol.

Es ist ebenfalls vorteilhaft, den Zubereitungen im Sinne der vorliegenden Erfindung übliche Antioxidantien zuzufügen. Es können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend der Aminosäuren (z. B. Glycin, Histidin, Tyrosin, Tryptophan) und deren Derivate-, Imidazole (z. B. Urocaninsäure ) und deren Derivate, Peptide wie DL-Camosin, D-Carnosin, L-Camosin und deren Derivate (z. B. Anserin), (Carotinoide, Carotine (z. B. [alpha]-Carotin, [beta]-Carotin, Lycopin) und deren Derivate, (Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, Linoleylester sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfuximin) in sehr geringen verträglichen Dosierungen (z. B. pmol bis j.Qnol/kg), ferner (Metall)-Chelatoren (z. B. [alpha]-Hydroxyfettsäuren, Palmitinsäure, Phytinsäure, Lactoferrin), [alpha]-Hydroxysäuren (z. B. Citronensäure, Milchsäure, Apfelsäure, Fumarsäure), Huminsäuren, Gallensäure, Gallenextrakte, Bilirubin, Biliverdin, EDTA, EGTA und deren Derivate, ungesättigte Fettsäuren und deren Derivate (z. B. gamma-Linolensäure, Linolsäure, Ölsäure), Folsäure und deren Derivate, Furfurylidensorbitol und dessen Derivate, Ubichinon und Ubichinol und deren Derivate-, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat oder -acetat) sowie Koniferylbenzoat des Benzoeharzes, Niacin und Niacinamid, Vitamine der B-Gruppe, Biotin, Rutinsäure und deren Derivate, [alpha]-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink. und dessen Derivate (z. B. ZnO, ZnSO₄, organische Komplexe/Salze z.B. Zn-PCA, Zn-Lactat, Zn-Tartrat) Selen und dessen Derivate (z. B. Selenmethionin), Stilbene und deren Derivate (z. B. Stilbenoxid, TransStilbenoxid) und geeignete Derivate (Salze, Ester, Ether, Zucker, Nukleotide, Nuklcoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05-20 Gew.-%, insbesondere 1-10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentration aus dem Bereich von 0,001-10 Gew.-%, bezogen auf das Gesamtgewicht der Formulierung, zu wählen. Zubereitungen gemäss der vorliegenden Erfindung können auch Verwendung als Grundlage für kosmetische oder dermatologische Desodorantien bzw. Antitranspirantien finden. Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber. beispielsweise die in der Patentoffenlegungsscbrift DE-P 4009 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäuren.

Keimhemmende Mittel sind ebenfalls geeignet, in die Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-'Trichlorcarbanilid, quaternierte Ammoniumverbindungen, Nelkenöl, Minzöl, Thymianöl, Triethylcitrat, Farnesol (3,7,11-Trimethyl-2,6,10-dodecatrien-I-ol) sowie die in den Patentoffenlegungsschriften DB-37 40 186, DE-39 381 40, DE-42 04 321, DE-42 29 707, DE-43 09 372, DB-44 11 664, DE-195 41 967, DE. 195 43 695, DE-195 43 696, DE-195 47 160, DE-196 02 108, DE-196 02 110, DB-196 02 111, DE.196 31 003, DE. 196 31 004 und DE-196 34019 und den Patentschriften DB 42 29 737, DE-42 37 081, DB 43 24 219, DE-44 294 67, DE-44 23 410 und DE-195 16 705 beschriebenen Wirkstoffe bzw. Wirkstoffkombinationen. Auch Natriumhydrogencarbonat ist vorteilhaft zu verwenden.

Die Menge solcher Wirkstoffe (eine oder mehrere Verbindungen) in den Zubereitungen beträgt vorzugsweise 0,001 bis 30 Gew.-%, besonders bevorzugt 0,05-20 Gew.-%, insbesondere 1-10 Gew.-%, bezogen auf das Gesamtgewicht der Zubereitung.

Die Wasserphase der kosmetischen Zubereitungen im Sinne der vorliegenden Erfindung kann auch Gelcharakter aufweisen, die neben einem wirksamen Gehalt an erfindungsgemäss eingesetzten Substanzen und dafür üblicherweise verwendeten Lösungsmitteln, bevorzugt Wasser, noch weitere organische Verdickungsmittel, z. B. Gummiarabicum, Xanthangummi, Natriumalginat, Stärke und Stärkederivate (z. B. Distärkephosphat), Cellulose, Cellulose-Derivate, vorzugsweise Methylcellulose, Hydroxymethylcelulose, Hydroxyethylcellulose, Hydroxypropylcellulose. Hydroxypropylmethylcellulose oder anorganische Verdickungmittel, z. B. Aluminiumsilikate, wie beispielsweise organisch modifizierte oder auch unmodifizierte Hectorite, Bentonite, oder dergleichen, oder ein Gemisch aus Polyethylenglykol und Polyethylenglykolstearat oder -distearat, enthalten. Das Verdickungsmittel ist in dem Gel z. B. in einer Menge zwischen 0, 1 und 30 Gew.-%, bevorzugt zwischen 0,5 und 15 Gew.-%, enthalten.

Ferner kann es von Vorteil sein. Zubereitungen gemäss der Erfindung grenz- bzw. oberflächenaktive Agentien zuzufügen, beispielsweise kationische Emulgatoren wie insbesondere quaternäre Tenside.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind Alkylbetain, Alkylamidopropylbetain und Alkyl-amidopropylhydroxysulfain. Die erfindungsgemäss verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidinium Chlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft sind auch kationische Polymere (z. B. Jaguare C162 [Hydroxypropyl Guar Hydroxypropyltrimonium Chloride] bzw. modifizierte Magnesiumaluminiumsilikaten (z. B. Quatemium-18-Hectorit, welches z. B. unter der Handelsbezeichnung Bentone 38 bei der Firma Rheox erhältlich ist, oder Stearalkonium Hectorit, welches z. B. unter der Handelsbezeichnung Softisane Gel bei der Hüls AG erhältlich ist), einzusetzen.

Die Zubereitungen können vorteilhaft auch Ölverdickungsmittel enthalten, um die taktilen Eigenschaften der Emulsion zu verbessern. Vorteilhafte Ölverdickungsmittel im Sinne der vorliegenden Erfindung sind beispielsweise weitere Feststoffe, wie z. B. hydrophobe Siliciumoxide des Typs Aerosil., welche von der Degussa AG erhältlich sind. Vorteilhafte Aerosiltypen sind beispielsweise Aerosil OX50, Aerosil 130, Aerosil 150, Aerosil 200, Aerosil 300, Aerosil 380, Aerosil MOX 80. Aerosil MOX 170, Aerosil COK 84, Aerosil R 202, Aerosil R 805, Aerosil R 812, Aerosil R 972, Aerosil R 974 und/oder Aerosil R976.

Ferner sind auch sogenannte Metallseifen (d. h. die Salze höherer Fettsäuren mit Ausnahme der Alkalisalze) vorteilhafte Verdickungsmittel im Sinne der vorliegenden Erfindung, wie beispielsweise Aluminium-Stearat, Zink-Stearat und/oder Magnesium-Stearat.

Ebenfalls vorteilhaft ist, den Zubereitungen amphotere bzw. zwitterionische Tenside (z. B. Cocoamidopylbetain) und Moisturizer (z. B. Betain) zuzusetzen. Vorteilhaft zu verwendende amphotere Tenside sind beispielsweise Acyl-/dialkylethylendiamin, beispielsweise Natriumacylamphoacetal, Dinatriumacylamphodipropionat, Dinatriumalkylamphodiacetat, Natriumacylamphohydroxypropylsulfonat, Dinatriumacylamphodiacetat und Natriumacylamphopropionat, N-Alkylaminosäuuren, beispielsweise Aminopropylalkylglutamid, Alkylaminopropionsäure, Natriumalkylimidodipropionat und Lauroamphocarboxyglycinat.

Die erfindungsgemäß verwendete kosmetische Zusammensetzung kann auf die zu behandelnden Hautstellen aufgetragen werden. Beim Auftragen auf die kosmetisch zu behandelnden Hautstellen wird die Zusammensetzung je nach Größe und Beschaffenheit der zu behandelnden Fläche beispielweise in Form einer Salbe, einer Creme, eines Gels, eines Schaums oder einer Lösung aufgetragen beziehungsweise aufgesprüht.

Die kosmetische Behandlung kann auch im Rahmen der gewöhnlichen Haut- und Körperpflege zum Einsatz kommen. Hierfür kann die erfindungsgemäß verwendete kosmetische Zusammensetzung beispielsweise als Bestandteil einer Gesichts- oder Körpercreme, einer Gesichtsmaske, eines Pflegeöls, einer Seife, eines Waschgels, einer Waschlotion oder eines Badezusatzes Verwendung finden. Je nach Art und Beschaffenheit der zu behandelnden Körperstelle kann diese auch in eine Lösung der kosmetischen Zusammensatzung in verdünnter oder unverdünnter Form über eine festgelegte Zeit getaucht bzw. gebadet werden.

Die erfindungsgemäß verwendete Zusammensetzung kann zur kosmetischen Behandlung von Hauterscheinungen, die keine therapeutische Behandlung erfordern, verwendet werden. Beispielsweise kann die Zusammensetzung zur Pflege von trockener oder rissiger Haut eingesetzt werden. Weiterhin kann die Ausstrahlung der Haut verbessert werden. Die erfindungsgemäß verwendete Zusammensetzung verleiht der Haut ein frisches und natürliches Aussehen, da das Hautbild verfeinert wird.

Mit zunehmendem Alter wird die Epidermis dünner und das Zellwachstum und die Zellteilung erfolgen langsamer. Dies bedeutet, dass sich die Epidermis nicht alle 27 Tage wie bei junger Haut, sondern häufig erst alle 40-60 Tage erneuert. Die Haut wird allgemein trockener, und es bilden sich Falten. Gegen Falten sind seit einigen Jahren spezielle Wirkstoffe, sogenannte Alpha-Hydroxysäuren oder Fruchtsäuren (AHA), auf dem Markt, die die abgestorbenen Hautschuppen der obersten Hautschicht (Epidermis) durch eine Art chemisches Peeling entfernen. Dadurch wird die oberste Hautschicht dünner und die Haut erscheint glatter und straffer. Eine glattere Haut kann jedoch auch durch eine Verstärkung der Proliferation von Keratinozyten hervorgerufen werden. Eine verstärkte Neubildung der obersten Hautschicht, wie sie durch die erfindungsgemäße Anwendung der Zusammensetzung erfolgt, wirkt dem Entstehen von Falten entgegen und reduziert die Faltentiefe von bereits bestehenden Falten.

Ein weiteres Einsatzgebiet ist die kosmetische Behandlung von Akne, insbesondere von Akne vulgaris. Die Etiologie der Akne vulgaris ist vielschichtig, jedoch spielen die folgenden vier Faktoren bei der Entstehung dieser Krankheit eine entscheidende Rolle.

Vermehrte Talgausschüttung: In der Pubertät beginnt der Körper Geschlechtshormone zu produzieren und insbesondere steigt der Testosteronspeigel an. Dieses Hormon wird durch das hauteigene Enzym, DH5 alpha-Reduktase, in das um das 10fach potentere Dihydrotestosteron umgewandelt. Außerdem können die Talgdrüsen gegenüber diesen Hormonen empfindlicher werden. Unter dem Einfluss dieser Hormone vergrößern sich dann die Talgdrüsen und es findet eine gesteigerte Talgproduktion statt.

Verhomungsstörungen: Eine erhöhte Vermehrung der Hornzellen (Hyperkeratose), die vermehrte Talgabsonderung und eine gestörte Abschilferung der Hornzellen sorgt für die Bildung von Konglomeraten und anschließend eines Hompfropfs (Komedo), der den Ausfuhrgang blockiert. Als Folge kann der Talg nicht mehr abfließen, und es bilden sich offene und geschlossene Komedone.

Vermehrung der Hautmikroflora (z.B. *Propionibacterium acnes):* Das Mikromilieu in den Komedonen und die erhöhte Talgproduktion, die ein hervorragender Nährboden für Mikroorganismen sind, begünstigen die Vermehrung der Aknebakterien.

Entzündungsreaktionen: Die Propionibakterien produzieren Enzyme (Lipasen), die wiederum die Fettsäuren des Talgs in freie Fettsäuren umwandeln. Diese Faktoren und weitere entzündungsfördernde und chemotaktisch wirksame Substanzen lösen Entzündungsreaktionen aus. Weiterhin scheinen reaktive Sauerstoffspezies (Reactive oxygen species (ROS)) bei den entzündlichen Prozessen eine Rolle zu spielen.

In der Kosmetik werden Zusammensetzungen angeboten, die einen dieser Faktoren gezielt ansprechen. Beispielsweise ist Salicylsäure altbekannt als Wirkstoff, der abnorme Verhomungen minimiert. Ohne sich auf eine Theorie festlegen zu wollen, wird davon ausgegangen, dass auch die erfindungsgemäß verwendete Zusammensetzung die orthologe Verhornung der Haut normalisiert und somit zu einer wesentlichen Verbesserung von aknegeplagter Haut beitragen kann.

Die erfindungsgemäß verwendete Zusammensetzung trägt wesentlich zur Stabilisierung des physiologischen pH-Wertes der Haut bei.

Die folgenden Beispiele erläutern die Erfindung.

### Beispiele

### 1. Zytotoxizität

Das zytotoxische Potential der NMDA-Rezeptor-Agonisten auf Keratinozyten wird untersucht. Dies erfolgt colorimetrisch mit der Kristallviolettmethode nach Gillies (Gillies et al., Anal. Biochem. 1986, 159, 109-113) in Analogie zu den darin dargestellten Untersuchungen für L-Glutamat. Hierfür werden normal humane epidermale Keratinozyten (NHEK) in 96-Wellplatten gezogen und für 24h und 48h mit den jeweiligen Effektoren inkubiert. Die vitalen, d.h. gefärbten Zellen werden bei 620 nm quantifiziert und die Werte mit der Negativkontrolle (nur Medium) und der Positivkontrolle (Ouabain) verglichen. Die Ergebnisse sind in Figur 1 dargestellt.

### 2. Untersuchungen zur Stimulation des keratinozytären NMDA-Rezeptors

Die Wirksamkeit der NMDA-Rezeptor-Agonisten auf den keratinozytären NMDA-Rezeptor wird über die Messung der intrazellulären Calciumkonzentration bei NHEK realisiert. Die Untersuchung erfolgt als Fluoreszenzmessung modifiziert nach Lipp und Niggli (Niggli et al.; Cell Calcium 1993, 14, 359-372). Als Fluoreszenzfarbstoffe werden Fura Red plus Fluo-3 verwandt. Die Messungen erfolgen mit einem Mikroplattenreader. Als Vergleichswert wird der Calciuminflux durch den "Standard"-Agonisten NMDA herangezogen. Die Spezifität der Ergebnisse wird durch die Applikation des selektiven NMDA-Rezeptor-Antagonisten AP5 (DL-2-amino-5-phosphonovaleric acid) geprüft.

In den Untersuchungsschritten 1 und 2 werden zunächst die optimalen Konzentrationen für die Einzelsubstanzen ermittelt. Diese optimal wirksamen, gleichzeitig aber nichttoxischen Konzentrationen werden dann in Kombination hinsichtlich ihrer Zytotoxizität und Wirksamkeit auf den keratinozytären NMDA-Rezeptor untersucht. Die so gefundene optimale Kombination der Effektoren wird dann bei den nachfolgend dargestellten Untersuchungen eingesetzt.

### 3. Untersuchungen zur Proliferation

Eine etwaige proliferationsfördernde bzw. -hemmende Eigenschaft der physiologischen NMDA-Rezeptor-Agonisten wird nicht-radioaktiv gemessen. Hierfür werden NHEK in 96-Wellplatten gezogen und für 24h und 48h mit dem Gemisch der Effektoren inkubiert. Die Messung der Proliferationsbeeinflussung erfolgt dann per ELISA-Technik mit BrdU (Bromodeoxyuridin). Hierbei wird der Einbau von BrdU in die DNA quantifiziert, wodurch Rückschlüsse auf die Proliferation der Zellen möglich sind. Hierfür stehen kommerziell verfügbare Kits zur Verfügung (z. B. BrdU Cell Proliferation Assay; Chemicon).

### 4. Untersuchungen zur Differenzierung

Für Untersuchungen zur differenzierungsfördemden Wirkung der physiologischen NMDA-Rezeptor-Agonisten werden NHEK in 60mm Kulturschalen gezogen. Dem Medium wird permanent die Agonisten-Kombination zugesetzt. Eine parallel angesetzte Kontrolle bleibt unbehandelt und fungiert als Zeitgeber. Zum Zeitpunkt, an dem die Kontrolle eine Konfluenz von 30%, 60% bzw. 100% erreicht hat, werden die Zellen geerntet und auf die Expression von folgenden Proliferations- und Differenzierungsmarkem untersucht.
- Transglutaminase Typ I (Schlüsselenzym der keratinozytären Differenzierung)
- Filaggrin (Differenzierungsmarker)
- Involucrin (Differenzierungsmarker)
- Cytokeratin 10 (Differenzierungsmarker)

Die Expression der genannten Marker wird per Westemblot-Technik gemessen. Ergänzend zu den Westernblot-Untersuchungen werden die Zellen mit diesen Antikörpern immunhistochemisch gefärbt und per digitaler Bildanalyse (vorhandene Software: Image Processing Tool Kit) vermessen.

### 5. Untersuchungen zur Proteinbiosynthese

Zur Untermauerung der breiten, die Physiologie des Keratinozyten vielfältig unterstützenden Anwendung der NMDA-Rezeptor-Agonisten soll ihr Einfluss auf die Proteinbiosynthese orientierend geprüft werden. Hierzu werden die Zellen direkt in Scintillatorgläschen kultiviert. Die Messung der Proteinsynthese erfolgt über den Einbau 3H-markierten Glutamats (ca. 30000 dpm/ml Medium), welches dem Kulturmedium zugegeben wird. Durch diverse Waschschritte und Trichloressigsäure-Fällung werden die neu synthetisierten Proteine von der radioaktiven Aminosäure getrennt. Nach Zugabe von Scintillatorflüssigkeit werden die Proben in einem Scintillationsmeßgerät vermessen. Parallel dazu wird die Proteinmenge bestimmt und schließlich die Menge des eingebauten Glutamats pro Proteinmenge ermittelt. In gleicher Weise kann mit 3H-markiertem Glycin und L-Aspartat gearbeitet werden.

## Patentansprüche

1. Verwendung einer Zusammensetzung, enthaltend mindestens einen N-Methyl-D-Aspartat-Rezeptor-Agonisten, zur kosmetischen Behandlung der menschlichen Haut.

2. Verwendung nach Anspruch 1, wobei der N-Methyl-D-Aspartat-Rezeptor-Agonist aus der Gruppe natürlich vorkommender Agonisten, bestehend aus L-Glutamat, L-Aspartat, N-Methyl-D-Aspartat, einem Chinolat, einem Homocysteat, D-Aspartat, D-Glutamat, trans-1-Amino-cyclobutan-1,3-dicarbonsäure, (±)-1-Aminocyclopentan-cis-1,3-dicarbonsäure, 2-Carboxy-3-carboxymethylchinolin, L-Cystein-sulfinsäure, 3-Carboxy-methyl-pyridine-2-carbonsäure, α-Amino-(3-hydroxy-5- i-soxazolyl)essigsäure, 4-Methoxy-7-nitroindolinyl-caged-L-glutamat, (S)-α-Amino-2,3-dihydro-4-methoxy-7-nitro-o-oxo-1H-indole-1-pentansäure, Pyridin-2,3-dicarbonsäure, (RS)-(Tetrazol-5-yl)glycin und Derivaten davon, gewählt wird.

3. Verwendung nach Anspruch 1 oder 2, wobei die Zusammensetzung zusätzlich mindestens einen N-Methyl-D-Aspartat-Rezeptor-Co-Agonisten enthält.

4. Verwendung nach Anspruch 3, wobei der N-Methyl-D-Aspartat-Rezeptor-Co-Agonist aus der Gruppe, bestehend aus Glycin, D-Serin, L-Alanin, D-Cycloserin, R(+)-3-amino-1-hydroxypyrrolid-2-on, R(+)-cis-β-methyl-3-amino-1-hydroxypyrrolid-2-on, einem Polyamin, und Derivaten davon, gewählt wird.

5. Verwendung nach einem der Ansprüche 1 bis 4, wobei die Zusammensetzung zusätzlich mindestens ein Zn²⁺-chelatisierendes Agens enthält.

6. Verwendung nach Anspruch 5, wobei das Zn²⁺-chelatisierende Agens aus der Gruppe, bestehend aus Dithiotreithol, Glutathion, CaEDTA, und Derivaten davon, gewählt wird.

7. Verwendung nach einem der Ansprüche 1 bis 6, wobei die Zusammensetzung zusätzlich mindestens ein Ca²⁺-lonen spendendes Agens enthält.
